# EUROPEAN PATENT APPLICATION

(11) **EP 3 656 472 A1**
(43) Date of publication of application: **27.05.2020**
(21) Application number: 18207377.5
(22) Date of filing: 20.11.2018
(51) Int. Cl.: B01L 3/00, G01N 35/08, G01N 33/48

(54) **CELL ANALYSER**

(71) Applicant: Lightcast Discovery Limited, Cambridge CB3 0FA (GB)
(72) Inventor: The designation of the inventor has not yet been filed
(74) Representative: Stratagem IPM Limited

(57) **Abstract**

A microdroplet-based device for analysing cells contained within a biological sample from for example a human or other mammalian patient is provided. It is characterised by the following parts:
• a sorting component comprised for separating cell-containing microdroplets from empty ones into a population of cell-containing first microdroplets;
• a microdroplet manipulation component for subsequently manipulating the first microdroplets using real or virtual electrowetting electrodes and including:
▪ a first zone including a means for introducing a reporter system into each first microdroplet by microdroplet merging;
▪ an second zone located within or adjacent the first zone in which merged microdroplets are thereafter detected in one or more detection windows and
▪ optionally a third zone in which microdroplets can be sub-divided and isolated for later recovery from the instrument

• an optical detection system for detecting an optical signal from the merged microdroplets arising from an interaction between the reporter system and the cells or an expressed product thereof selected from a brightfield microscope, a darkfield microscope, a means for detecting chemiluminescence, a means for detecting Forster resonance energy transfer or a means for detecting fluorescence.

## Description

This invention relates to a device for the rapid identification, manipulation and selection of cells. It is especially useful for the manipulation of mammalian cells, either from immortalised cell culture samples or directly from tissue samples. It is also especially useful for the rapid and parallel screening of patient samples thought to contain evidence of one or more microbial infections.

Devices for manipulating droplets or magnetic beads have been previously described in the art; see for example US6565727, US20130233425 and US20150027889. In the case of droplets, this outcome may be typically achieved by causing the droplets, for example in the presence of an immiscible carrier fluid, to travel through a microfluidic space defined by two opposed walls of a cartridge or microfluidic tubing. Embedded within one or both of these walls are microelectrodes covered with a dielectric layer each of which is connected to an A/C biasing circuit capable of being switched on and off rapidly at intervals to modify the electric field characteristics of the layer. This gives rise to localised directional capillary forces in the vicinity of the microelectrodes which can be used to steer the droplet along one or more predetermined pathways. Such devices, which employ what hereinafter and in connection with the present invention will be referred to as 'real' electrowetting electrodes, are known in the art by the acronym EWOD (Electrowetting on Dielectric) devices.

A variant of this approach, in which the electrowetting forces are optically-mediated, known in the art as optoelectrowetting and hereinafter the corresponding acronym OEWOD, has been disclosed in, for example, US20030224528, US20150298125, US20160158748, US20160160259 and Applied Physics Letters 93 221110 (2008). In particular, the first of these three patent applications discloses various microfluidic devices which include a microfluidic cavity defined by first and second walls and wherein the first wall is of composite design and comprised of substrate, photoconductive and insulating (dielectric) layers. In this single-sided embodiment, between the photoconductive and insulating layers is disposed an array of conductive cells which are electrically isolated from one another and coupled to the photoactive layer and whose functions are to generate corresponding electrowetting electrode locations on the insulating layer. At these locations, the surface tension properties of the droplets can be modified by means of an electrowetting field as described above. These conductive cells may then be temporarily switched on by light impinging on the photoconductive layer. This approach has the advantage that switching is made much easier and quicker although its utility is to some extent still limited by the arrangement of the electrodes. Furthermore, there is a limitation as to the speed at which droplets can be moved and the extent to which the actual droplet pathway can be varied.

Double-sided embodiments of this latter approach has been disclosed in University of California at Berkeley thesis UCB/EECS-2015-119 by Pei. In one example, a cell is described which allows the manipulation of relatively large droplets in the size range 100-500µm using optoelectrowetting across a surface of Teflon AF deposited over a dielectric layer using a light-pattern over electrically-biased amorphous silicon. However, in the devices exemplified the dielectric layer is thin (100nm) and only disposed on the wall bearing the photoactive layer.

Recently, in our pending application EP17177204.9 we have described a device for manipulating microdroplets which uses optoelectrowetting to provide the motive force. In this OEWOD device, the microdroplets are translocated through a microfluidic space defined by containing walls; for example a pair of parallel plates having the microfluidic space sandwiched therebetween. At least one of the containing walls includes what are hereinafter referred to as 'virtual' electrowetting electrodes locations which are generated by selectively illuminating an area of a semiconductor layer buried within. By selective illumination of the layer with light from a separate light source, a virtual pathway of virtual electrowetting electrode locations can be generated transiently along which the microdroplets can be caused to move. In our corresponding application EP17180391.9, use of this device as an operative part of a nucleic acid sequencer is described.

We have now applied this technique to develop an analyser which is particularly suitable for the manipulation and detection of cells in a biological sample; for example one taken by a medical practitioner in a hospital or surgery, or one used in research assays to elucidate and isolate the functional behaviour of cells. Thus, according to the present invention there is provided a device for analysing cells contained within a biological sample characterised by comprising:
- a sorting component for separating cell-containing microdroplets from empty ones into a population of cell-containing first microdroplets;
- a microdroplet manipulation component for subsequently manipulating the first microdroplets using real or virtual electrowetting electrodes and including:
   ▪ a first zone including a means for introducing a reporter system into each first microdroplet by means of microdroplet merging;
   ▪ a second zone located within or adjacent the first zone in which merged microdroplets are thereafter detected in one or more detection windows and
   ▪ optionally a third zone in which microdroplets can be sub-divided and isolated for later recovery from the instrument and
- an optical detection system for detecting an optical signal from the merged microdroplets arising from an interaction between the reporter system and the cells or an expressed product thereof selected from a brightfield microscope, a darkfield microscope, a means for detecting chemiluminescence, a means for detecting Forster resonance energy transfer or a means for detecting fluorescence.

The sorting component employed in the device of the invention is a means for separating microdroplets containing one or more cells (hereinafter 'filled microdroplets') from a larger population some of which are empty. Depending on the type of sorting component chosen, the microdroplets are directed down or towards one of two different microfluidic pathways or receiving locations depending on whether they are filled or empty. In some embodiments, access to one or other of these is controlled by a divider or actuation of an electromechanical gate acting in response to the analysis of each microdroplet in an analytical window. In another preferred embodiment, sorting is accomplished by applying a responding optically-mediated electrowetting force in the analytical window to a stream of the microdroplets so that the chosen ones are pulled into a holding area or array. The rejected microdroplets then remain in the stream and thereafter can be discarded. In another, preferred embodiment, the sorting decision is based on an optical phenomenon; doe example brightfield microscopy or by detecting an optical property associated with the cells e.g. the presence of a fluorescent tag or marker. In another embodiment, sorting may be achieved by a dielectrophoretic method in which a temporary electrical field is applied in the analytical window to deflect each microdroplet in turn towards one of two different pathways either side of a divider. In one preferred embodiment, the sorting component comprises a first microfluidic channel terminating in analytical chamber; at least two second microfluidic channels connected to the analytical chamber on the downstream side at least one of which carries away the first microdroplets, a light source for illuminating the analytical chamber; a brightfield microscope or fluorescence detector for obtaining data from each illuminated microdroplet in the analytical chamber; at least one OEWOD structure operable to direct the microdroplets down one of the two second channels and a microprocessor adapted to operate the structure(s) in response to a result from an identification algorithm applied to data received from the microscope or fluorescence analyser.

In one embodiment, the device further comprises a cell culturing component which is either an integral component of the device itself or separately located before or after the sorting component; preferably after the sorting component. Here, the microdroplets are held whilst any cells contained within are cultured so as to stimulate cell division and growth. Suitably, the cell-culturing component comprises a vessel in which the microdroplets are held under optimal culturing conditions; typically from 6 to 72 hours at a temperature in the range above 25°C (e.g. 25 to 40°C) and an inlet port for introducing the microdroplets thereinto. In one embodiment, the cell-culturing component further comprises a thermostatically-controlled heater or heating jacket and optionally a timer which controls filling and discharge cycles for the vessel. In one embodiment, the contents of the vessel comprise an emulsion of microdroplets in an immiscible carrier fluid and the vessel further comprises inlet and outlet ports through which carrier fluid can be passed thereby allowing it to be replaced over time. In another embodiment, applicable to a corresponding method to be used with the device the immiscible carrier fluid is a hydrocarbon or silicone oil; preferably a fluorocarbon oil such as HFE7500, HFE7700 or FC-40. Such oils may suitably further contain surfactants and other additives to maintain microdroplet stability and low levels of the nutrients and those gases required to maintain cell growth. These can be used to replenish the contents of the microdroplets by interfacial diffusion. Alternatively, aqueous microdroplets containing only essential growth media can also be introduced into the vessel via, for example the same or an additional inlet port and thereafter caused to merge with the first microdroplets.

In one embodiment, of particular utility where the weight ratio of aqueous microdroplets to oil is low, there is a tendency for the microdroplets to shrink over time as they translocate through the device; a phenomenon which can lead to a loss of reactivity within them. One preferred way of counteracting this effect is to use an oil which has been hydrated. Since generally the oils described above do not have a high capacity for dissolving water, hydration is suitably achieved by creating micelles or secondary microdroplets of water or aqueous buffer within the oil phase. This buffer may have a composition which is the same as or different to that of the microdroplets themselves. In one embodiments these micelles or secondary microdroplet may contain up to five times the salt content of the microdroplets themselves and optionally contain glycerol. Typically these micelles and secondary microdroplets are an order of magnitude smaller.

In one embodiment, the vessel further comprises a surface provided with a plurality of locations at which the microdroplets can be positioned and agitated to stir their contents using optically mediated electrowetting forces. In another embodiment, these locations comprise an OEWOD structure such as one of those described herein and a source of rapidly flashing rotational light having a circumferential pathway around the periphery of each location. In another embodiment, the movement of the light source may be one or more side-to-side motions. In one manifestation, these locations are defined by circles having a diameter of up to 100 microns. In one corresponding embodiment, the vessel comprises a dielectric-coated composite first wall including a semiconductor layer, an A/C biasing means and a transparent second wall located opposite though which electromagnetic radiation, can be used to illuminate the first wall at the desired location(s).

The device of the invention may further comprise a sample preparation component either integral with the device itself or separately located before the sorting component. It suitably comprises a means for creating an emulsion of the microdroplets in the immiscible carrier fluid from an aliquot of the biological sample. In one embodiment, this component includes a means for severing microdroplets from the biological sample into the carrier fluid which comprises at least one location where an electrowetting stretching force is applied to the biological sample. In one preferred embodiment this severing means comprises:
- a first electrowetting location adapted to receive the biological sample;
- at least one second electrowetting location arranged so that the first and second electrowetting electrode locations define a pathway along which microdroplets severed from the sample can be transported using directional electrowetting forces;
- an AC drive circuit arranged at the first electrowetting location and comprised of either an electrode and an associated AC electrical circuit or a semiconductor zone activated by the impingement of electromagnetic light thereon and
- a DC charging circuit arranged at the first electrowetting location and adapted to electrostatically charge the surface of the biological sample.

In one embodiment, the severing means further comprises a control circuit for switching between the drive and charging circuits which are suitably AC and DC circuits respectively. In another embodiment, the severing means further comprises an analyser for analysing the contents of each microdroplet produced from the biological sample. In this respect, the biological sample can be in the form of any aqueous material such as blood, plasma, sputum, urine or aqueous material derived from tissue biopsies. Further information about suitable severing means can be found in our co-pending application EP18201162.7 to which the reader is directed.

Turning to the microdroplet manipulation component which is used to subsequently manipulate the first microdroplets produced by the sorting component, this is suitably a microfluidic chip comprised of a first zone, a second zone and an optical detection system some of which include real or virtual electrowetting electrode locations and some of which are linked together by one or more microfluidic pathways along which the first microdroplets are driven by pneumatic and/or electrowetting forces. Suitably, the electrowetting electrodes are virtual and established at locations in one or more OEWOD structures. In one preferred embodiment of the chip these OEWOD structures are comprised of:
- a first composite wall comprised of:
   ▪ a first transparent substrate
   ▪ a first transparent conductor layer on the substrate having a thickness in the range 70 to 250nm;
   ▪ a photoactive layer activated by electromagnetic radiation in the wavelength range 400-1000nm on the conductor layer having a thickness in the range 300-1000nm and
   ▪ a first dielectric layer on the conductor layer having a thickness in the range 120 to 160nm;
- a second composite wall comprised of:
   ▪ a second substrate;
   ▪ a second conductor layer on the substrate having a thickness in the range 70 to 250nm and
   ▪ optionally a second dielectric layer on the conductor layer having a thickness in the range 120 to 160nm
   wherein the exposed surfaces of the first and second dielectric layers are disposed at least 10µm apart to define a microfluidic space adapted to contain microdroplets;
- an A/C source to provide a voltage across the first and second composite walls connecting the first and second conductor layers;
- at least one source of second electromagnetic radiation having an energy higher than the bandgap of the photoexcitable layer adapted to impinge on the photoactive layer to induce corresponding virtual electrowetting electrode locations on the surface of the first dielectric layer and
- means for manipulating the points of impingement of the electromagnetic radiation on the photoactive layer so as to vary the disposition of the virtual electrowetting electrode locations thereby creating at least one optically-mediated electrowetting pathway along which the microdroplets may be caused to move.

In one embodiment, the first and second walls of the structures are transparent with the microfluidic space sandwiched in-between. In another, the first substrate and first conductor layer are transparent enabling light from the source of electromagnetic radiation (for example multiple laser beams or LED diodes) to impinge on the photoactive layer. In another, the second substrate, second conductor layer and second dielectric layer are transparent so that the same objective can be obtained. In yet another embodiment, all these layers are transparent.

Suitably, the first and second substrates are made of a material which is mechanically strong for example glass metal or an engineering plastic. In one embodiment, the substrates may have a degree of flexibility. In yet another embodiment, the first and second substrates have a thickness in the range 100-1000µm.

The first and second conductor layers are located on one surface of the first and second substrates and are typically have a thickness in the range 70 to 250nm, preferably 70 to 150nm. In one embodiment, at least one of these layers is made of a transparent conductive material such as Indium Tin Oxide (ITO), a very thin film of conductive metal such as silver or a conducting polymer such as PEDOT or the like. these layers may be formed as a continuous sheet or a series of discrete structures such as wires. Alternatively, the conductor layer may be a mesh of conductive material with the electromagnetic radiation being directed between the interstices of the mesh.

The photoactive layer is suitably comprised of a semiconductor material which can generate localised areas of charge in response to stimulation by the source of the second electromagnetic radiation. Examples include hydrogenated amorphous silicon layers having a thickness in the range 300 to 1000nm. In one embodiment, the photoactive layer is activated by the use of visible light.

The photoactive layer in the case of the first wall and optionally the conducting layer in the case of the second wall are coated with a dielectric layer which is typically in the thickness range from 120 to 160nm. The dielectric properties of this layer preferably include a high dielectric strength of >10^7 V/m and a dielectric constant of >3. Preferably, it is as thin as possible consistent with avoiding dielectric breakdown. In one embodiment, the dielectric layer is selected from alumina, silica, hafnia or a thin non-conducting polymer film.

In another embodiment of the structures, at least the first dielectric layer, preferably both, are coated with an anti-fouling layer to assist in the establishing the desired microdroplet/carrier fluid/surface contact angle at the various virtual electrowetting electrode locations, and additionally to prevent the contents of the microdroplets adhering to the surface and being diminished as the microdroplet is moved through the chip. If the second wall does not comprise a second dielectric layer, then the second anti-fouling layer may be applied directly onto the second conductor layer. For optimum performance, the anti-fouling layer should assist in establishing a microdroplet/carrier fluid/surface contact angle that should be in the range 70-110° when measured as an air-liquid-surface three-point interface at 25°C. In one embodiment, these layer(s) have a thickness of less than 50nm and are typically a monomolecular layer. In another, these layers are comprised of a polymer of an acrylate ester such as methyl methacrylate or a derivative thereof substituted with hydrophilic groups; e.g. alkoxysilyl. Preferably, either or both of the anti-fouling layers are hydrophobic to ensure optimum performance. In certain embodiments there is an interstitial layer of silica interposed between the anti-fouling layer and the dielectric layer in order to form a chemically compatible interface between the layers, such a layer is typically less than 10nm thick.

The first and second dielectric layers, and therefore the first and second walls, define a microfluidic space which is at least 10µm in width and in which the microdroplets are contained. Preferably, before they are contained, the microdroplets themselves have an intrinsic diameter which is more than 10% greater, suitably more than 20% greater, than the width of the microdroplet space. By this means, on entering the chip the microdroplets are caused to undergo compression leading to enhanced electrowetting performance through e.g. a better microdroplet merging capability.

In one embodiment the first and second dielectric layers are coated with an antifouling coating such as fluorosilane.

In another embodiment the first and second dielectric layers are coated with a biocompatible coating such as (3-Aminopropyl)trimethoxysilane.

In another embodiment, the microfluidic space includes one or more spacers for holding the first and second walls apart by a predetermined amount. Options for spacers includes beads or pillars, ridges created from an intermediate resist layer which has been produced by photopatterning. Various spacer geometries can be used to form narrow channels, tapered channels or partially enclosed channels which are defined by lines of pillars. By careful design, it is possible to use these spacers to aid in the deformation of the microdroplets, subsequently perform microdroplet splitting and effect operations on the deformed microdroplets. The same spacers can be used to guide the flow of fluids in the microfluidic space when filling, priming and emptying the device.

The first and second walls are biased using a source of A/C power attached to the conductor layers to provide a voltage potential difference therebetween; suitably in the range 10 to 150 volts.

These preferred OEWOD structures are typically employed in association with a source of second electromagnetic radiation having a wavelength in the range 400-1000nm and an energy higher than the bandgap of the photoexcitable layer. Suitably, the photoactive layer will be activated at the virtual electrowetting electrode locations where the incident intensity of the radiation employed is in the range 0.01 to 0.2 Wcm⁻². The source of electromagnetic radiation is, in one embodiment, highly attenuated and in another pixellated so as to produce corresponding photoexcited regions on the photoactive layer which are also pixellated. By this means, pixellated virtual electrowetting electrode locations are induced on the first dielectric layer.

Where the source of electromagnetic radiation is pixellated it is suitably supplied either directly or indirectly using a reflective screen such as a digital micromirror device (DMD) illuminated by light from LEDs or other lamps. This enables highly complex patterns of virtual electrowetting electrode locations to be rapidly created and destroyed on the first dielectric layer thereby enabling the microdroplets to be precisely steered along essentially any virtual pathway using closely-controlled electrowetting forces. This is also especially advantageous where there is a requirement for the chip to manipulate many thousands of such microdroplets simultaneously along multiple pathways. Such electrowetting pathways can be viewed as being constructed from a continuum of virtual electrowetting electrode locations on the first dielectric layer.

The points of impingement of the sources of electromagnetic radiation on the photoactive layer can be any convenient shape including the conventional circular and annulus. In one embodiment, the morphologies of these points are determined by the morphologies of the corresponding pixelations and in another correspond wholly or partially to the morphologies of the microdroplets once they have entered the microfluidic space. In one preferred embodiment, the points of impingement and hence the electrowetting electrode locations may be crescent-shaped and orientated in the intended direction of travel of the microdroplet. Suitably the electrowetting electrode locations themselves are smaller than the microdroplet surface adhering to the first wall and give a maximal field intensity gradient across the contact line formed between the droplet and the surface dielectric. In one embodiment of the OEWOD structure, the second wall also includes a photoactive layer which enables virtual electrowetting electrode locations to also be induced on the second dielectric layer by means of the same or different source of electromagnetic radiation. The addition of a second dielectric layer enables transition of the wetting edge of a given microdroplet from the upper to the lower surface of the structure, and the application of more electrowetting force to each microdroplet.

The first zone which forms part of the microdroplet manipulation component or chip suitably comprises, in one embodiment, a holding reservoir comprising an inlet port for introducing the first microdroplets and an outlet port attached by an electrowetting pathway to the second zone. The first zone further includes a port (e.g. the first inlet port) for introducing a reporter system which in one suitable embodiment is a second inlet port for introducing second aqueous microdroplets containing a reporter system designed to identify the nature of the cells contained in the first microdroplets. In one embodiment, the reservoir further includes an array of locations where the first microdroplets can be held using inter alia electrowetting forces whilst the second microdroplets are driven over them; in the process causing a degree of merging of the first and second microdroplets. The merged first/second microdroplets (hereinafter 'merged microdroplets') can then be held at the locations until the reporter system has interacted sufficiently with cells to subsequently generate an optimum optical signal at which time they are transported to the second zone by electrowetting. In one preferred embodiment, a single zone encompassing the duties of both the first and second zones is employed; for example by detecting the merged microdroplets at the merging locations referred to above.

Suitable reporter systems which can be introduced into the first microdroplets can in principle be any system which is characteristic of or which may be used to assay the presence of a given cell type in biological sample. Such reporter systems include, for example, a reporter gene, a cell-surface biomarker or a reporter molecule selective for an enzyme, protein or antibody expressed by cells of the cell type being sought. A related class of reporter system can be a second reporter cell which responds to the presence of relevant material expressed by the cell being sought. Many such assays are known in the prior art and suitable candidates for use herein will in many cases be readily apparent to one of ordinary skill. Furthermore it will be appreciated that by introducing a plurality of different reporter systems into the first microdroplets the device may be used in a multiplexed way so as to carry out parallel and simultaneous searches for a range different cell types associated with a range of candidate infections.

The second zone is suitably comprised of one or more detection windows through which the merged microdroplets can be interrogated and thus analysed using an optical detection system. In one embodiment this second zone is a transparent region of the chip. In another embodiment, the optical detection system is one designed to detect an optical signal arising from an interaction between the reporter system and the cells searched for or an expressed product thereof; for example a protein or enzyme. Suitably, the optical detection system is selected from a brightfield microscope, a darkfield microscope, a means for detecting chemiluminescence, a means for detecting Forster resonance energy transfer or a means for detecting fluorescence. In one embodiment, the detection system also includes a source of light to illuminate the merged microdroplets and/or a microprocessor for receiving a signal from one of the detectors and providing assay results to a user in the form of, for example, a visual display or count. In one embodiment, the microprocessor is further adapted by means of a feedback loop to control one or more of the performance of the sorting component; the rate of introduction of the first microdroplets into the first zone and the rate of merging of the first and reporter system-containing microdroplets in response to a signal detected by the optical detection system.

A particular advantage of an instrument using oEWOD structures for performing the droplet manipulations is that an optical addressing system focused on the sample is built in to the instrument. By multiplexing and de-multiplexing the excitation and emission light required for optical detection in with the illumination required for oEWOD control, it is possible to consolidate many of the optical functions into one simpler and lower cost assembly. For example, it is possible to de-multiplex luminescence emission from the assembly using a long-pass dichroic mirror to divert light from the manipulation column and to a high-sensitivity detection camera. Another embodiment uses two dichroic mirrors; a first mirror to multiplex in fluorescence excitation light from a lamp and a second to de-multiplex fluorescence emission. For embodiments requiring more sophisticated illumination schemes such as time-resolved Forster resonance energy transfer it is favourable to employ the same structured illumination system which addresses the oEWOD manipulation patterns to apply a time-dependent and spatially varying illumination pattern. As well as using dichroic mirrors for these multiplexing operations it is possible to use elements such as dispersive filters, dispersive lenses or gratings. For some applications it is favourable to perform temporal multiplexing whereby the structured illumination system is used to switch rapidly between excitation sources.

Optionally, the device further comprises a third zone for recovering microdroplets from the first and/or second zones Here, the first microdroplets can be sub-divided and isolated for later recovery from the instrument; for example for more detailed or and a confirmatory analysis. In one embodiment, the third zone is comprised of one or more outlet ports from the second zone connected to a temporary storage vessel. By replenishing the first and second zones using the oEWOD transport mechanism, it is possible to perform many recoveries of microdroplets in sequence, enabling multiple droplets to be separately recovered from one port.

As well as the optically mediated manipulation of fluids in the OEWOD structure, the instrument may also include a network of pumps and valves to manipulate the flow inside the device by selective application of hydraulic pressure to the various inlet and outlet ports. Preferably, this network comprises two-position valves connected to each outlet and a set of pressure sources (such as pumps), collection vessels and reservoirs connected to the same valves. By changing the configuration of each valve, it is possible to apply positive or negative pressure within the device via the reservoirs and collection vessels and hence cause the flow of material into, out of or within the device.

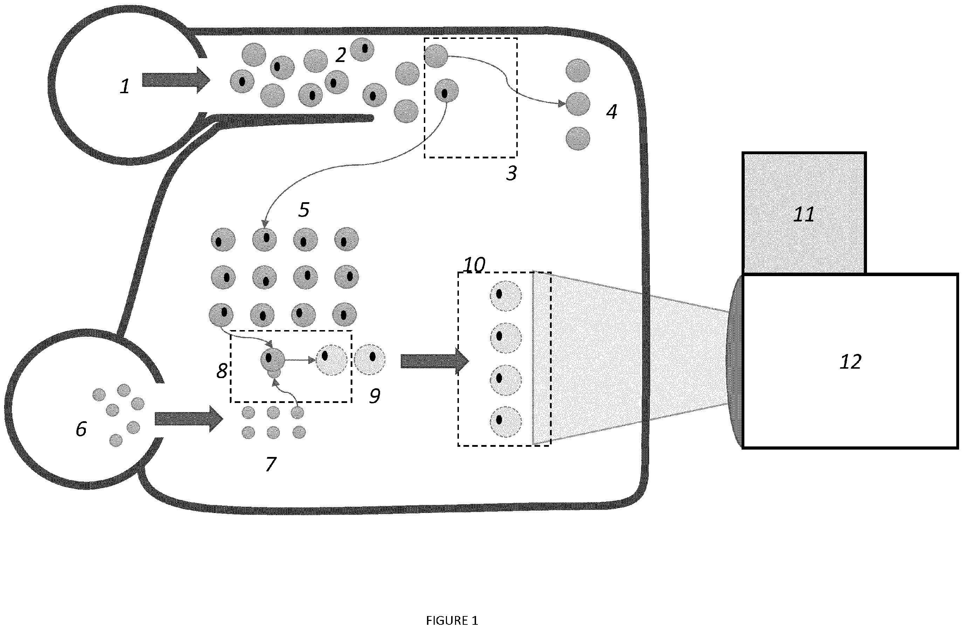

The device is now illustrated with reference to Figure 1
A fluid inlet (1) admits an emulsion (2) of cell-containing microdroplets in a fluorocarbon oil. These microdroplets are then transferred by means of OEWOD structures (not shown) to a sorting zone (3) where they are optically sorted into those which are empty (4) and those which contain cells (5). Thereafter each of (5) are transferred to merging zone (8) also by means of OEWOD structures where they are held for a defined period of time under conditions which promote cell growth and division within each. At the end of this period, second inlet (6) admits microdroplets containing a fluorescence reporter system selective for a cell type of interest (7) which are then merged with (5) at (8) to form a new merged microdroplets(9). (9) are transferred by means of OEWOD structures to optical window (10) where a fluorescence signal characteristic of the reporter system is detected using an optical detection instrument (11) comprised of an LED light source, a photodetector and a microprocessor. (11) is partially multiplexed with optical manipulation projector (12).

## Claims

1. A device for analysing cells contained within a biological sample **characterised by** comprising:
• a sorting component for separating cell-containing microdroplets from empty ones into a population of cell-containing first microdroplets;
• a microdroplet manipulation component for subsequently manipulating the first microdroplets using real or virtual electrowetting electrodes and including:
• a first zone including a means for introducing a reporter system into each first microdroplet by means of microdroplet merging;
• a second zone located within or adjacent the first zone in which merged microdroplets are thereafter detected in one or more detection windows and
• optionally a third zone in which microdroplets can be sub-divided and isolated for later recovery from the instrument and
• an optical detection system for detecting an optical signal from the merged microdroplets arising from an interaction between the reporter system and the cells or an expressed product thereof selected from a brightfield microscope, a darkfield microscope, a means for detecting chemiluminescence, a means for detecting Forster resonance energy transfer or a means for detecting fluorescence.

2. A device as claimed in claim 1 **characterised by** further comprising a cell-culturing component either integral with the device or separately located before or after the sorting component in which the microdroplets are held whilst any cells contained within are cultured.

3. A device as claimed in claim 1 or claim 2 **characterised by** further comprising a sample preparation component either integral with the device or separately located before the sorting component which comprises a means for creating an emulsion of the microdroplets in an immiscible carrier fluid from the biological sample.

4. A device as claimed in claim 3 **characterised in that** the sample preparation component includes a means for severing microdroplets from the biological sample into the carrier fluid which includes at least one location where an electrowetting stretching force is applied to the biological sample.

5. A device as claimed in any one of the preceding claims **characterised in that** the optical detection means comprises a source of first electromagnetic radiation adapted to impinge on the first microdroplets in the detector window(s) and a detector for detecting fluorescence emitted therefrom.

6. A device as claimed in any one of the preceding claims **characterised in that** the microdroplet manipulation component includes one or more OEWOD structures comprised of:
▪ a first composite wall comprised of
• a first transparent substrate
• a first transparent conductor layer on the substrate having a thickness in the range 70 to 250nm;
• a photoactive layer activated by electromagnetic radiation in the wavelength range 400-1000nm on the conductor layer having a thickness in the range 300-1000nm and
• a first dielectric layer on the conductor layer having a thickness in the range 120 to 160nm;
▪ a second composite wall comprised of
• a second substrate;
• a second conductor layer on the substrate having a thickness in the range 70 to 250nm and
• a second dielectric layer on the conductor layer having a thickness in the range 120 to 1600nm
wherein the exposed surfaces of the first and second dielectric layers are disposed less than 50µm apart to define a microfluidic space adapted to contain microdroplets;
▪ an A/C source to provide a voltage across the first and second composite walls connecting the first and second conductor layers;
▪ at least one source of second electromagnetic radiation having an energy higher than the bandgap of the photoexcitable layer adapted to impinge on the photoactive layer to induce corresponding virtual electrowetting electrodes locations on the surface of the first dielectric layer;
▪ means for manipulating the points of impingement of the electromagnetic radiation on the photoactive layer so as to vary the disposition of the virtual electrowetting electrode locations thereby creating at least one optically-mediated electrowetting pathway along which the first microdroplets may be caused to move.

7. A device as claimed in any one of the preceding claims **characterised in that** first zone includes a reservoir comprising an array of first microdroplet-holding sites, a port for introducing second microdroplets containing the reporter system and a means for driving the second microdroplets across the first microdroplet-holding sites so that first and second microdroplets are caused to merge.

8. A device as claimed in claim 7 **characterised in that** the means for driving the second microdroplets across the first microdroplet-holding sites comprises one or more pathways of real or virtual electrowetting electrodes.

9. A device as claimed in claim 7 **characterised in that** a region of the microfluidic connected to an inlet filling port is used to load droplets under continuous hydraulic flow, and that an overlapping OEWOD region is used to remove droplets from this continuous flow region in to a separate region of the chip for further operations

10. A device as claimed in any one of the preceding claims **characterised in that** the cell-culturing component further comprises a means to agitate the contents of each microdroplet using optically-mediated electrowetting forces.

11. A device as claimed in any one of the preceding claims **characterised in that** the device is provided with a heater and temperature controller to control the temperature of the microdroplets in the cell-culturing component within the range 25 to 40°C.

12. A device as claimed in any one of the preceding claims **characterised in that** at least one of the sorting component, the cell-culturing component and the sample preparation component is provided with electrowetting electrode locations to enable the droplets to be manipulated therein and/or therebetween.

13. A device as claimed in any one of the preceding claims **characterised in that** the dielectric surfaces of the microdroplet manipulation component are provided with an anti-fouling coating.

14. A device as claimed in any one of the preceding claims **characterised by** further comprising a microprocessor adapted by means of a feedback loop to control one or more of the performance of the sorting component; the rate of introduction of the first microdroplets into the first zone and the rate of merging of the first and second microdroplets in response to a signal supplied by the detection system.

15. A device as claimed in any one of the preceding claims comprising an optical assembly to illuminate the optical electrowetting device which further comprises a set of auxiliary optical components for the purpose of detecting signals characterising cells which are partially multiplexed within the assembly.
